# EUROPEAN PATENT APPLICATION

(11) **EP 4 647 085 A1**
(43) Date of publication of application: **12.11.2025**
(21) Application number: 24738668.3
(22) Date of filing: 02.01.2024
(51) Int. Cl.: A61K 48/00, A61K 31/713, A61P 25/28, G01N 33/50, C12Q 1/6883, A23L 33/13

(54) **USE OF FGFR3 EXPRESSION OR ACTIVITY INHIBITOR FOR TREATING NEURODEGENERATIVE DISEASES**

(30) Priority: 03.01.2023 KR 20230000696
(71) Applicant: Seoul National University R & DB Foundation, Seoul 08826 (KR)
(72) Inventor: MOOK, In Hee, Seoul 08826 (KR); SUH, Kyu Jin, Seoul 08826 (KR); KIM, Dong Kyu, Seoul 08826 (KR)
(74) Representative: Schwarz & Partner Patentanwälte GmbH
(86) International application number: PCT/KR2024/000034
(87) International publication number: WO 2024/147597

(57) **Abstract**

In the present invention, it is identified that FGFR3 can be used as a novel target for neurodegenerative disease treatment, and an agent for preventing and treating neurodegenerative diseases is provided on the basis of the identification. In addition, provided is a method for screening for candidates for prevention and treatment of neurodegenerative diseases. According to a screening method of the present invention, novel candidates capable of preventing and/or treating neurodegenerative diseases can be effectively selected, and substances selected according to the screening method of the present invention are used such that a pharmaceutical composition for preventing and treating neurodegenerative diseases can be provided.

## Description

### [Technical Field]

The present application claims priority to Korean Patent Application No. 10-2023-0000696, filed on January 3, 2023, and the entire disclosure of the above application is incorporated herein by reference.

The present invention relates to the use of an FGFR3 (fibroblast growth factor receptor 3) inhibitor for treating neurodegenerative diseases, and more specifically, to a composition for preventing, improving, or treating neurodegenerative diseases comprising an inhibitor of expression or activity of FGFR3 as an active ingredient, and a method for screening the same.

### [Background of the Invention]

Alzheimer's disease (AD) is the most common degenerative brain disease that causes dementia, and it is a progressive neurodegenerative disease characterized by neuronal loss and cognitive impairment. Alzheimer's disease exhibits neuropathological features of extracellular amyloid-β (Aβ) plaques and intracellular neurofibrillary tangles. Amyloid-β is generated by the sequential cleavage of amyloid-β precursor protein (APP) mediated by BACE1 (Beta-secretase 1) and presenilin/γ-secretase. The production and aggregation of amyloid-β play a critical role in triggering complex pathological processes that lead to neurodegeneration, neurofibrillary tangles, inflammation, and neuronal loss.

The activation of microglia, which indicates neuroinflammation, is another pathological characteristic observed in the brains of Alzheimer's disease patients. Furthermore, microglia play a potential pathogenic role in many central nervous system (CNS) diseases, including chronic neurodegenerative disorders such as AD, PD (Parkinson's disease), HIV (human immunodeficiency virus)-associated dementia, and multiple sclerosis. Activated microglia and leukocytes produce various inflammatory mediators, including anaphylatoxin complement, cytokines, chemokines, and prostaglandins.

Meanwhile, another pathological feature of Alzheimer's disease is the presence of extracellular senile plaques composed of neurofibrillary tangles (hereinafter abbreviated as 'NFT') formed by abnormally hyperphosphorylated tau.

The tau protein consists of four parts: the N-terminal projection domain, the proline-rich domain, the microtubule-binding domain, and the C-terminal domain. It is known that when tau is abnormally hyperphosphorylated or modified in the neurons of the central nervous system, it causes degenerative neurological diseases such as tauopathy. Representative tauopathies include Alzheimer's disease dementia, Pick's disease, and frontotemporal dementia with parkinsonism linked to chromosome 17 (FTDP-17).

Among the two pathogenic proteins that play the most critical roles in the pathological process of Alzheimer's disease, amyloid-β and tau, Aducanumab, which targets amyloid-β, is the only approved drug for Alzheimer's disease. However, no treatment targeting tau protein, one of the two major pathogenic proteins of Alzheimer's disease, has been approved to date. Therefore, the proposal of a new therapeutic target capable of blocking tau protein pathology can provide an important option for the development of treatments for degenerative neurological diseases such as Alzheimer's disease.

### [Problem to be Solved]

Accordingly, the inventors of the present invention have conducted extensive research on the association between tau protein and Alzheimer's disease and on developing a novel therapeutic target capable of blocking tau protein pathology. As a result, we discovered that amyloid beta induces the internalization of the FGFR3 receptor in neuronal cells, during which tau protein is also introduced into the cells, leading to tau protein-induced pathological phenomena, thereby completing the present invention.

Therefore, an object of the present invention is to provide a pharmaceutical composition for preventing or treating a neurodegenerative disease, comprising an inhibitor of expression or activity of FGFR3 (fibroblast growth factor receptor 3) as an active ingredient.

Another object of the present invention is to provide a pharmaceutical composition for preventing or treating a neurodegenerative disease, consisting of an inhibitor of expression or activity of FGFR3 (fibroblast growth factor receptor 3).

An object of the present invention is to provide a pharmaceutical composition for preventing or treating a neurodegenerative disease, consisting essentially of an inhibitor of expression or activity of FGFR3 (fibroblast growth factor receptor 3).

Another object of the present invention is to provide a food composition for preventing or improving a neurodegenerative disease, comprising an inhibitor of expression or activity of FGFR3 (fibroblast growth factor receptor 3) as an active ingredient.

Another object of the present invention is to provide a food composition for preventing or improving a neurodegenerative disease, consisting of an inhibitor of expression or activity of FGFR3 (fibroblast growth factor receptor 3).

Another object of the present invention is to provide a food composition for preventing or improving a neurodegenerative disease, consisting essentially of an inhibitor of expression or activity of FGFR3 (fibroblast growth factor receptor 3).

Another object of the present invention is to provide a method for screening a candidate substance for preventing and treating a neurodegenerative disease, comprising the steps of:
(a) treating a test substance to cells expressing mRNA or protein of FGFR3;
(b) measuring the expression level of FGFR3 mRNA, the expression level of FGFR3 protein, or the activity of FGFR3 protein; and
(c) selecting a substance that decreases the expression level of FGFR3 mRNA, the expression level of FGFR3 protein, or the activity of FGFR3 protein compared to a control group (untreated group) as a candidate substance.

Another object of the present invention is to provide the use of an inhibitor of expression or activity of FGFR3 (fibroblast growth factor receptor 3) for manufacturing a composition for treating a neurodegenerative disease.

Another object of the present invention is to provide a method for treating a neurodegenerative disease, comprising administering a composition comprising an inhibitor of expression or activity of FGFR3 (fibroblast growth factor receptor 3) as an active ingredient to a subject in need thereof.

### [Means for Solving the Problem]

To achieve the aforementioned objectives of the present invention, the present invention provides a pharmaceutical composition for preventing or treating a neurodegenerative disease, comprising an inhibitor of expression or activity of FGFR3 (fibroblast growth factor receptor 3) as an active ingredient.

In addition, the present invention provides a pharmaceutical composition for preventing or treating a neurodegenerative disease, consisting of an inhibitor of expression or activity of FGFR3 (fibroblast growth factor receptor 3).

In addition, the present invention provides a pharmaceutical composition for preventing or treating a neurodegenerative disease, consisting essentially of an inhibitor of expression or activity of FGFR3 (fibroblast growth factor receptor 3).

To achieve another objective of the present invention, the present invention provides a food composition for preventing or improving a neurodegenerative disease, comprising an inhibitor of expression or activity of FGFR3 (fibroblast growth factor receptor 3) as an active ingredient.

In addition, the present invention provides a food composition for preventing or improving a neurodegenerative disease, consisting of an inhibitor of expression or activity of FGFR3 (fibroblast growth factor receptor 3).

In addition, the present invention provides a food composition for preventing or improving a neurodegenerative disease, consisting essentially of an inhibitor of expression or activity of FGFR3 (fibroblast growth factor receptor 3).

To achieve another objective of the present invention, the present invention provides a method for screening a candidate substance for preventing and treating a neurodegenerative disease, comprising the steps of:
(a) treating a test substance to cells expressing mRNA or protein of FGFR3;
(b) measuring the expression level of FGFR3 mRNA, the expression level of FGFR3 protein, or the activity of FGFR3 protein; and
(c) selecting a substance that decreases the expression level of FGFR3 mRNA, the expression level of FGFR3 protein, or the activity of FGFR3 protein compared to a control group (untreated group) as a candidate substance.

To achieve another objective of the present invention, the present invention provides the use of an inhibitor of expression or activity of FGFR3 (fibroblast growth factor receptor 3) for manufacturing a composition for treating a neurodegenerative disease.

To achieve another objective of the present invention, the present invention provides a method for treating a neurodegenerative disease, comprising administering a composition comprising an inhibitor of expression or activity of FGFR3 (fibroblast growth factor receptor 3) as an active ingredient to a subject in need thereof.

Hereinafter, the present invention will be described in detail.

The present invention provides a pharmaceutical composition for preventing or treating a neurodegenerative disease, comprising an inhibitor of expression or activity of FGFR3 (fibroblast growth factor receptor 3) as an active ingredient.

In the present invention, the "FGFR3" is one of the four members of the FGFR family of transmembrane tyrosine kinase receptors involved in intracellular signal transduction pathways. Activation of FGFR through interaction with fibroblast growth factors (FGFs) has been shown to play a critical role in both adult and embryonic development, with pleiotropic consequences including cell proliferation and survival, migration, differentiation, and growth arrest.

The gene of FGFR3 is known as Gene ID: 2261 (human), 14184 (mouse), and its mRNA reference sequences are known as NM_000142, NM_001163213, NM_022965, NM_001354809, NM_001354810 in humans, and NM_001163215, NM_001163216, NM_001163217, NM_001205270, NM_008010 in mice. Its protein reference sequences are known as NP_000133, NP_001156685, NP_075254, NP_001341738, NP_001341739 in humans.

In the present invention, "tau protein" is a microtubule-associated protein that is abundant in the central nervous system and is primarily produced by neurons. The main function of tau is to stabilize microtubules. Six tau isoforms exist in the adult brain; these tau isoforms are products of alternative splicing of a single gene. Tau protein achieves control of microtubule stability through isoforms and phosphorylation, and hyperphosphorylation of tau protein can cause the formation of twisted and straight filaments (neurofibrillary tangles), which contribute to the pathology of tauopathies such as Alzheimer's disease and other neurodegenerative diseases.

The "tauopathy" refers to a group of neurodegenerative diseases resulting from pathological aggregation of tau protein within so-called neurofibrillary tangles (NFTs) in the brain. Some examples of tauopathies include frontotemporal dementia (FTD), Alzheimer's disease, progressive supranuclear palsy, corticobasal degeneration, and frontotemporal lobar degeneration.

The "neurodegenerative disease" can be understood as a degenerative disease of the nervous system or a neuroinflammatory disease, which collectively refers to diseases in which specific groups of brain cells in the brain and cortex gradually lose their function and decrease in number due to unknown causes. Since the nerve cells in the brain and cortex perform very diverse functions depending on their location, the clinical manifestations vary greatly depending on which areas of nerve cells are first damaged and lose function, and how these dysfunctions progress. Neurodegenerative diseases exhibit abnormalities in a wide range of functions, including motor control, cognitive function, sensory function, and autonomic nervous functions that are autonomously regulated without our awareness, due to the reduction or loss of nerve cell function.

The most important and representative diseases among neurodegenerative diseases include Alzheimer's disease, frontotemporal dementia, frontotemporal lobar degeneration, corticobasal degeneration, progressive supranuclear palsy, Pick's disease, Parkinson's disease, or Huntington's disease.

In one embodiment, the inhibitor of FGFR3 expression may be siRNA (small interference RNA), shRNA (short hairpin RNA), miRNA (microRNA), ribozyme, DNAzyme, PNA (peptide nucleic acids), antisense oligonucleotide, or aptamer that complementarily binds to DNA or mRNA of the FGFR3 gene.

In one embodiment, the inhibitor of FGFR3 activity refers to a substance that directly or indirectly acts on FGFR3 to reduce or inhibit its activity and may be a compound, peptide, peptide mimetics, fusion protein, antibody, or aptamer that specifically binds to FGFR3 protein, but is not limited thereto.

In the present specification, the FGFR3 expression inhibitor refers to a substance that directly or indirectly acts on FGFR3 to reduce or inhibit its expression and may include siRNA (small interference RNA), shRNA (short hairpin RNA), miRNA (microRNA), ribozyme, DNAzyme, PNA (peptide nucleic acids), antisense oligonucleotide, or aptamer that complementarily binds to DNA or mRNA of the FGFR3 gene, but is not limited thereto.

The mechanism by which the substance inhibits the expression or activity of FGFR3 is not particularly limited and may act, for example, by inhibiting gene expression such as transcription or translation, or by converting the active form into an inactive form.

According to one embodiment of the present invention, when the expression or activity of FGFR3 is inhibited, the amount of hyperphosphorylated tau protein is significantly reduced, and at the same time, neuronal cell death is inhibited, resulting in an increase in the number of neuronal cells compared to the control group. In addition, it was confirmed that the amount of aggregated tau protein was significantly reduced in mice in which the FGFR3 gene was inhibited. Furthermore, it was confirmed that the amount of oligomeric forms of tau protein, known to be harmful to neurons, decreased when the expression of FGFR3 was inhibited.

In one aspect of the present invention, the inhibition of expression or activity of FGFR3 may be characterized as inhibition of expression or activity of the extracellular domain of FGFR3.

In one aspect of the present invention, the extracellular domain of FGFR3 may consist of the amino acid sequence of SEQ ID NO: 1.

[SEQ ID NO: 1]

MVVPACVLVFCVAVVAGATSEPPGPEQRVVRRAAEVPGPEPSQQEQVAFGSGDTVELSC
HPPGGAPTGPTVWAKDGTGLVASHRILVGPQRLQVLNASHEDAGVYSCQHRLTRRVLCH
FSVRVTDAPSSGDDEDGEDVAEDTGAPYWTRPERMDKKLLAVPAANTVRFRCPAAGNP
TPSISWLKNGKEFRGEHRIGGIKLRHQQWSLVMESVVPSDRGNYTCVVENKFGSIRQTY
TLDVLERSPHRPILQAGLPANQTAILGSDVEFHCKVYSDAQPHIQWLKHVEVNGSKVGP
DGTPYVTVLKSWISENVEADARLRLANVSERDGGEYLCRATNFIGVAEKAFWLRVHGP
QAAEEELMETDEAGSVYAGVLSYGVVFFLFILVVAAVILCR

In the present specification, "prevention" refers to any act of suppressing or delaying the onset of a neurodegenerative disease through the administration of the composition according to the present invention.

In the present specification, "treatment" refers to any act of improving or beneficially altering the symptoms of neurodegenerative diseases and complications thereof through the administration of the composition according to the present invention. Those skilled in the art to which the present invention pertains will be able to determine the exact criteria for diseases for which the composition of the present invention is effective, and assess the degree of improvement, enhancement, and treatment by referring to materials presented by organizations such as the Korean Medical Association.

In the present specification, "improvement" refers to an act of at least reducing parameters related to the condition being treated, for example, the severity of symptoms. At this time, the composition of the present invention may be used simultaneously with or separately from therapeutic agents for the prevention or improvement of neurodegenerative diseases.

The therapeutically effective amount of the composition of the present invention may vary depending on various factors, such as the method of administration, the target site, and the condition of the subject.

The pharmaceutical composition of the present invention is administered in a pharmaceutically effective amount. The term "pharmaceutically effective amount" as used in the present invention refers to an amount sufficient to treat the disease with a reasonable benefit/risk ratio applicable to medical treatment and without causing side effects. The effective dosage level may be determined based on factors such as the health condition of the subject, the type and severity of the neurodegenerative disease, the activity of the drug, the sensitivity to the drug, the method of administration, the time of administration, the route of administration, the excretion rate, the treatment period, the combination or simultaneous use of other drugs, and other factors well known in the medical field. The composition of the present invention may be administered as a single therapeutic agent or in combination with other therapeutic agents, and may be administered sequentially or simultaneously with conventional therapeutic agents, either as a single or multiple doses. Considering all the above factors, it is important to administer the minimum amount necessary to achieve maximum efficacy without side effects, which can be easily determined by those skilled in the art.

In one embodiment, the pharmaceutical composition may be in one or more formulations selected from the group consisting of oral formulations, topical agents, suppositories, sterile injectable solutions, and sprays.

The composition of the present invention may also include carriers, diluents, excipients, or combinations thereof, which are commonly used in biological preparations. Pharmaceutically acceptable carriers are not particularly limited as long as they are suitable for in vivo delivery of the composition. For example, compounds described in Merck Index, 13th ed., Merck & Co. Inc., saline, sterile water, Ringer's solution, buffered saline, dextrose solution, maltodextrin solution, glycerol, ethanol, and mixtures of one or more of these components may be used. If necessary, other conventional additives such as antioxidants, buffers, and bacteriostatic agents may be added. Additionally, diluents, dispersants, surfactants, binders, and lubricants may be added to formulate aqueous solutions, suspensions, emulsions, pills, capsules, granules, or tablets. Furthermore, the composition may be formulated in a preferred manner depending on the disease or component using appropriate methods in the art or methods disclosed in Remington's Pharmaceutical Science (Mack Publishing Company, Easton PA, 18th, 1990).

The composition of the present invention may further contain one or more active ingredients exhibiting the same or similar functions. The composition of the present invention comprises the active ingredient in an amount of 0.0001 to 10% by weight, preferably 0.001 to 1% by weight, based on the total weight of the composition.

The pharmaceutical composition of the present invention may further include pharmaceutically acceptable additives. Pharmaceutically acceptable additives may include starch, gelatinized starch, microcrystalline cellulose, lactose, povidone, colloidal silicon dioxide, dibasic calcium phosphate, lactose, mannitol, malt, gum arabic, pregelatinized starch, corn starch, powdered cellulose, hydroxypropyl cellulose, Opadry, sodium starch glycolate, carnauba wax, synthetic aluminum silicate, stearic acid, magnesium stearate, aluminum stearate, calcium stearate, sucrose, dextrose, sorbitol, and talc. The pharmaceutically acceptable additives according to the present invention are preferably included in an amount of 0.1 to 90 parts by weight relative to the composition, but are not limited thereto.

The composition of the present invention may be administered parenterally (e.g., intravenously, subcutaneously, intraperitoneally, or topically) or orally, with oral administration being most preferred. The dosage range may vary depending on factors such as the body weight, age, gender, health condition, diet, time of administration, method of administration, excretion rate, and severity of the disease.

Liquid formulations for oral administration of the composition of the present invention include suspensions, internal solutions, emulsions, and syrups, which may include commonly used simple diluents such as water and liquid paraffin, as well as various excipients such as wetting agents, sweeteners, flavoring agents, and preservatives. Formulations for parenteral administration include sterile aqueous solutions, non-aqueous solvents, suspensions, emulsions, freeze-dried preparations, and suppositories.

The present invention also provides a food composition for preventing or improving neurodegenerative diseases, comprising an inhibitor of expression or activity of FGFR3 (fibroblast growth factor receptor 3) as an active ingredient.

The food composition of the present invention includes all forms such as functional foods, nutritional supplements, health foods, and food additives.

The food composition of the above type can be manufactured in various forms by conventional methods known in the art. For example, health foods may be prepared in the form of tea, juice, and drinks containing the active ingredient, but are not limited thereto, and may be consumed in liquid, granulated, encapsulated, or powdered form. Additionally, the composition may be prepared in combination with known active ingredients effective for improving condition. Furthermore, functional foods may include, but are not limited to, beverages (including alcoholic beverages), fruits and their processed foods (e.g., canned fruits, bottled fruits, jams, marmalades, etc.), fish, meat and their processed foods (e.g., ham, sausage, corned beef, etc.), bread and noodles (e.g., udon, soba, ramen, spaghetti, macaroni, etc.), fruit juices, various drinks, cookies, taffy, dairy products (e.g., butter, cheese, etc.), edible vegetable oils, margarine, vegetable proteins, retort foods, frozen foods, and various seasonings (e.g., soybean paste, soy sauce, sauces, etc.), to which the active ingredient is added. Additionally, the active ingredient may be manufactured in powder or concentrate form for use as an additive.

The preferred content of the active ingredient in the food composition of the present invention is not limited thereto but is preferably 0.1 to 90% by weight in the final manufactured food. More preferably, the food composition of the present invention may be manufactured in the form of functional foods or dietary supplements, particularly in combination with active ingredients known to have preventive or improving effects on neurodegenerative diseases.

The present invention also provides a method for screening candidate substances for preventing and treating neurodegenerative diseases, comprising the following steps: (a) treating test substances to cells expressing mRNA or protein of FGFR3; (b) measuring the expression level of FGFR3 mRNA, the expression level of FGFR3 protein, or the activity of FGFR3 protein; and (c) selecting a substance that decreases the expression level of FGFR3 mRNA, the expression level of FGFR3 protein, or the activity of FGFR3 protein compared to a control group (untreated group) as a candidate substance.

The substance that reduces the activity of FGFR3 may be siRNA, shRNA, miRNA, ribozyme, DNAzyme, PNA (peptide nucleic acids), antisense oligonucleotide, peptide, an antibody specific to FGFR3 protein, aptamer, natural extract, or chemical substance, but is not limited thereto, and encompasses all substances used or to be used in the future for inhibiting the expression of genes and/or proteins in the art.

In the present invention, the measurement of the expression level of FGFR3 mRNA may be performed using RT-PCR, quantitative or semi-quantitative RT-PCR, quantitative or semi-quantitative real-time RT-PCR, northern blot, and DNA or RNA chip, but is not limited thereto.

In the present invention, the measurement of the expression level of FGFR3 protein may be performed using a method selected from the group consisting of western blot, ELISA, radioimmunoassay, radial immunodiffusion, Ouchterlony immunodiffusion, rocket immunoelectrophoresis, immunohistochemical staining, immunoprecipitation analysis, complement fixation analysis, FACS, and protein chip, but is not limited thereto.

In the present invention, after the step (c), a step of evaluating pharmacological activity in a suitable in vivo model may be further performed.

In addition, the present invention provides the use of an inhibitor of expression or activity of FGFR3 (fibroblast growth factor receptor 3) for manufacturing a composition for treating a neurodegenerative disease.

In addition, the present invention provides a method for treating a neurodegenerative disease, comprising administering a composition comprising an inhibitor of expression or activity of FGFR3 (fibroblast growth factor receptor 3) as an active ingredient to a subject in need thereof.

The term "effective amount" in the present invention refers to an amount that, when administered to a subject, exhibits an effect of improving, treating, detecting, diagnosing, or inhibiting or reducing a neurodegenerative disease, and the term "subject" may refer to an animal, preferably a mammal, particularly an animal including a human, and may also include cells, tissues, or organs derived from an animal. The subject may be a patient in need of the above effect.

The term "treatment" in the present invention comprehensively refers to improving a neurodegenerative disease or symptoms caused by the disease, and may include curing the disease, substantially preventing the disease, or improving the condition, and includes alleviating, curing, or preventing one or most symptoms caused by the disease, but is not limited thereto.

The term "comprising" as used herein is used in the same sense as "including" or "characterized by," and does not exclude additional components or steps of methods that are not specifically mentioned in the composition or method according to the present invention. The term "consisting of" means excluding additional elements, steps, or components not specifically described. The term "consisting essentially of" means that, within the scope of the composition or method, it may include substances or steps that do not substantially affect the basic characteristics of the described substances or steps.

### [Effect of the Invention]

The present invention reveals that FGFR3 can be used as a new target for the treatment of neurodegenerative diseases and, based on this, provides a preventive and therapeutic agent for neurodegenerative diseases. In addition, it provides a method for screening candidate substances for the prevention and treatment of neurodegenerative diseases. According to the screening method of the present invention, it is possible to effectively select new candidate substances capable of preventing and/or treating neurodegenerative diseases, and there is an effect of providing a pharmaceutical composition for the prevention and treatment of neurodegenerative diseases using the substances selected according to the screening method of the present invention.

### [Brief Description of the Drawings]

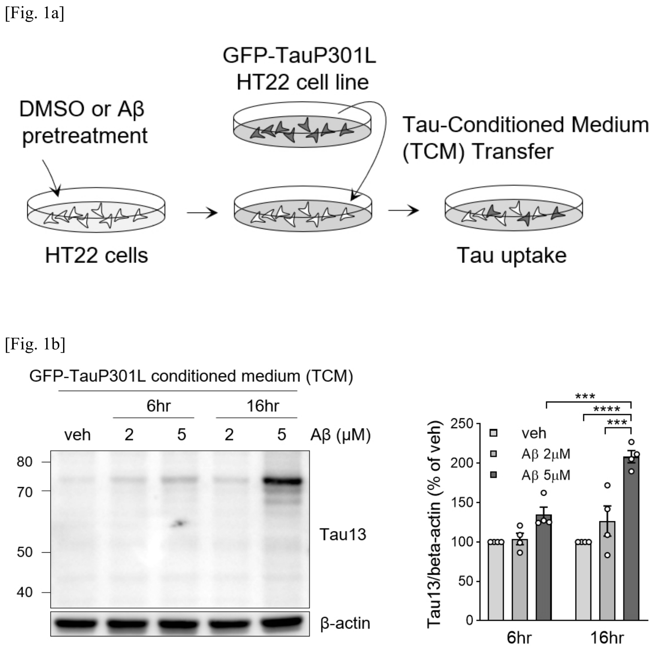

Fig. 1a and Fig. 1b show the results of treating a culture medium containing tau protein to neuronal cells pretreated with amyloid beta (Fig. 1a) and measuring the amount of intracellular tau protein according to the concentration and treatment time of amyloid beta (Fig. 1b).

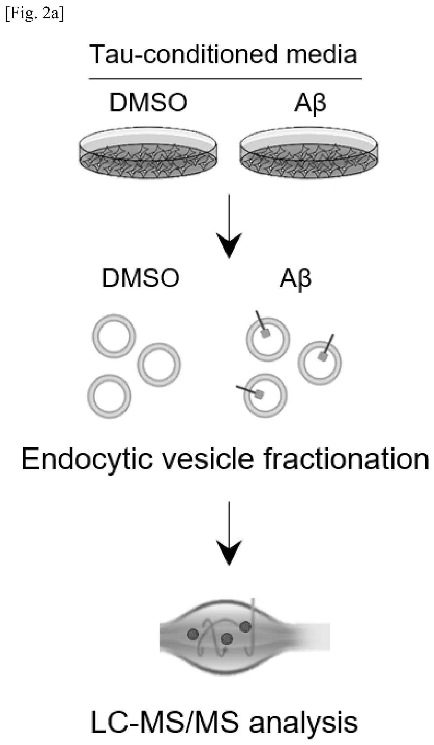

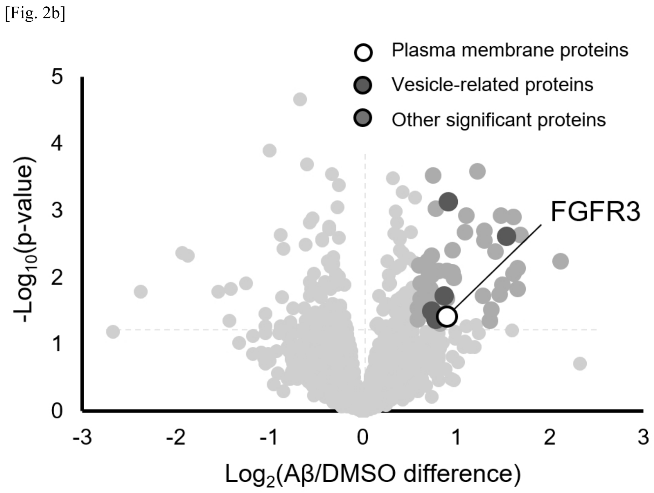

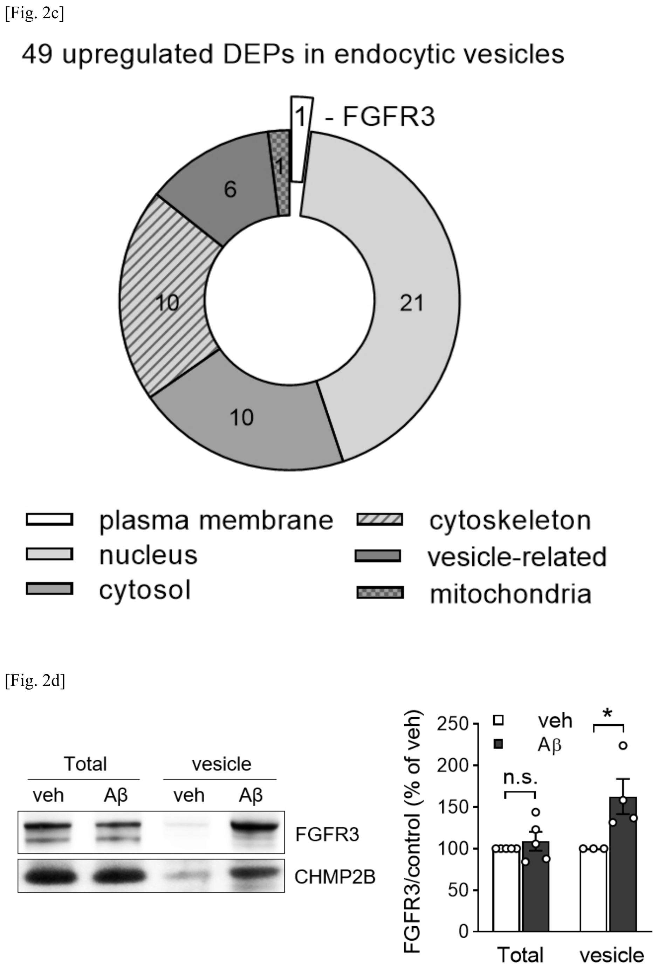

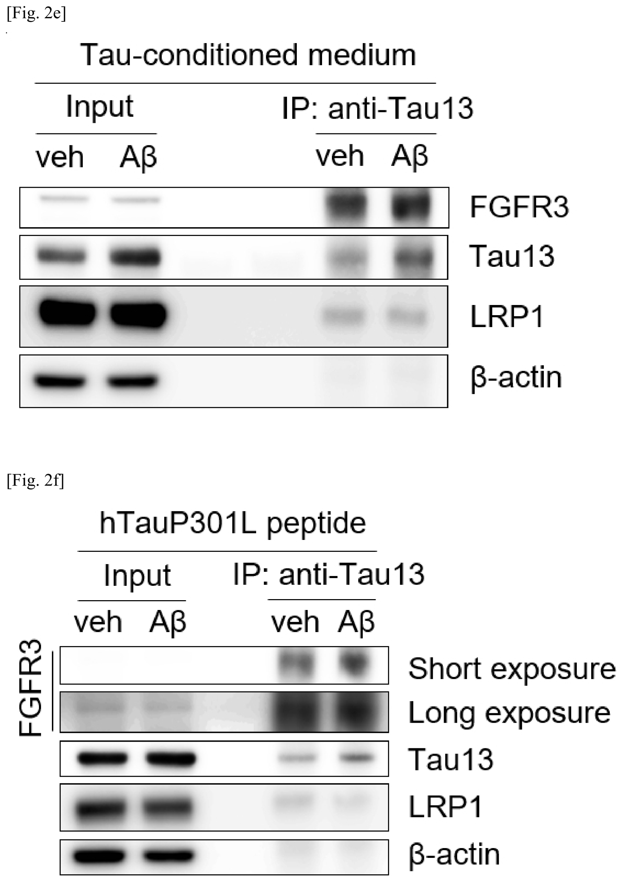

Figs. 2a to 2f show the results of identifying receptors affecting the internalization of tau protein when a culture medium containing tau protein is treated to neuronal cells pretreated with amyloid beta, through LC-MS/MS proteomic analysis (Figs. 2a to 2c), western blot (Fig. 2d), and immunoprecipitation (Figs. 2e and 2f).

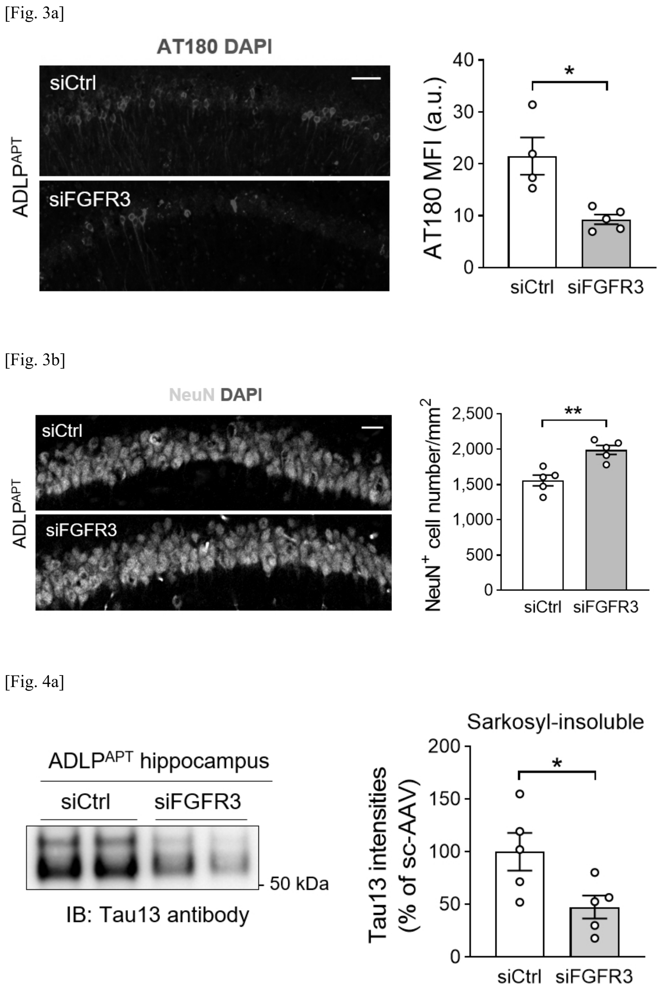

Figs. 3a and 3b show the results of injecting siRNA viruses of SEQ ID NOs: 2 to 5, which regulate the expression of FGFR3, into an Alzheimer's disease animal model overexpressing both amyloid beta and tau protein, thereby suppressing gene expression, reducing the expression of FGFR3 protein in neuronal cells in the brain, and then measuring the amount of hyperphosphorylated tau protein in the hippocampus (Fig. 3a) and confirming neuronal cell death (Fig. 3b).

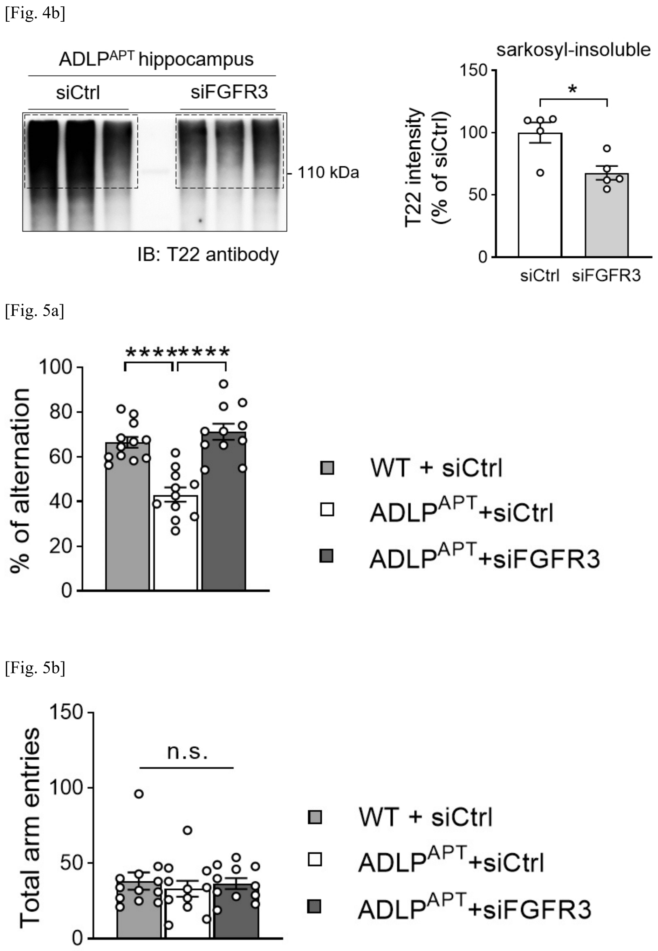

Figs. 4a and 4b show the results of injecting siRNA viruses of SEQ ID NOs: 2 to 5, which regulate the expression of FGFR3, into an Alzheimer's disease animal model overexpressing both amyloid beta and tau protein, thereby suppressing gene expression, reducing the expression of FGFR3 protein in neuronal cells in the brain, and then measuring the amount of aggregated tau protein (Fig. 4a) and the amount of oligomeric tau protein (Fig. 4b).

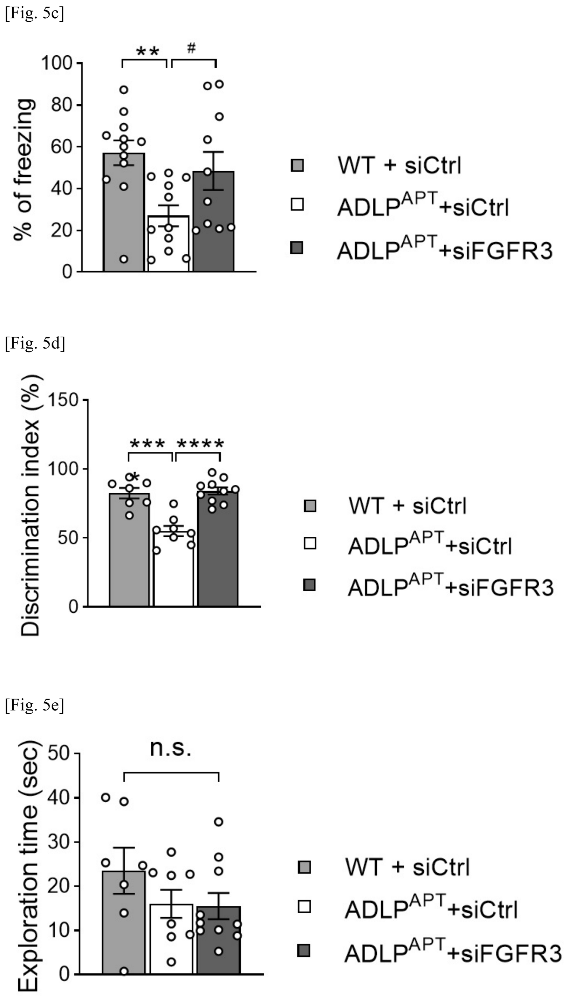

Figs. 5a to 5e show the results of injecting siRNA viruses of SEQ ID NOs: 2 to 5, which regulate the expression of FGFR3, into an Alzheimer's disease animal model overexpressing both amyloid beta and tau protein, thereby suppressing gene expression, and then performing a Y-maze test (Figs. 5a and 5b), a contextual fear conditioning test (Fig. 5c), and a novel object recognition test (Figs. 5d and 5e).

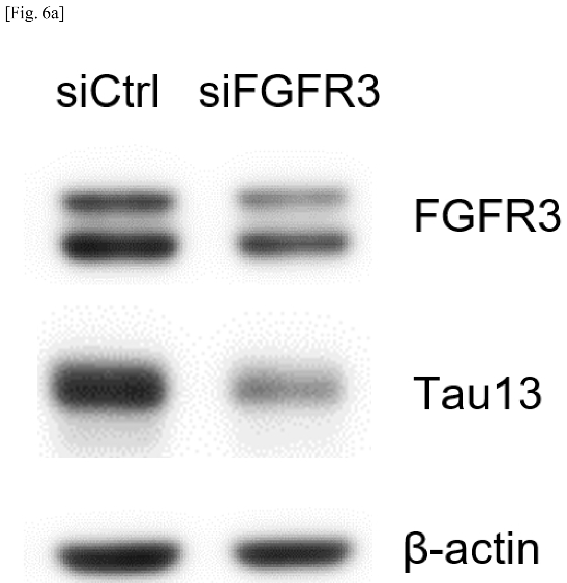

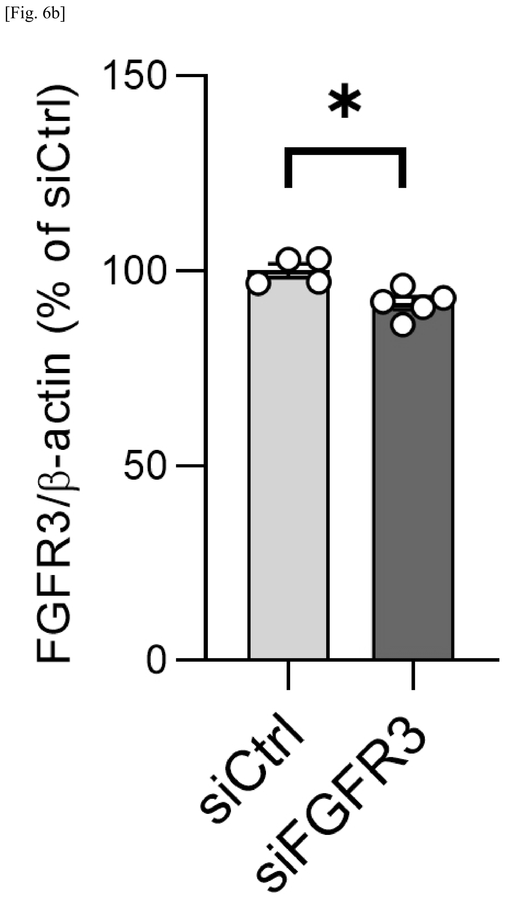

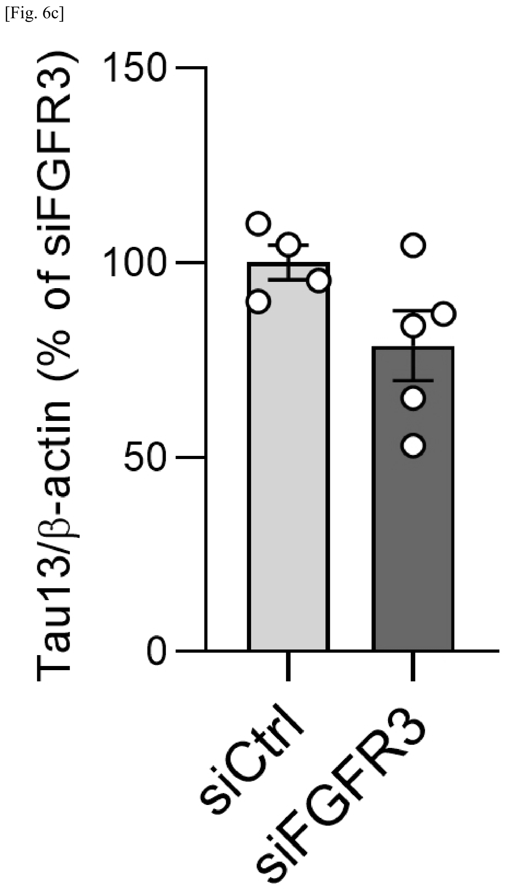

Figs. 6a to 6c show the results of infecting HT22 neuronal cells with siFGFR3 to suppress gene expression, and then evaluating the expression level of FGFR3 protein in the cells (Figs. 6a and 6b) and the amount of Tau13 protein absorbed into the cells (Figs. 6a and 6c).

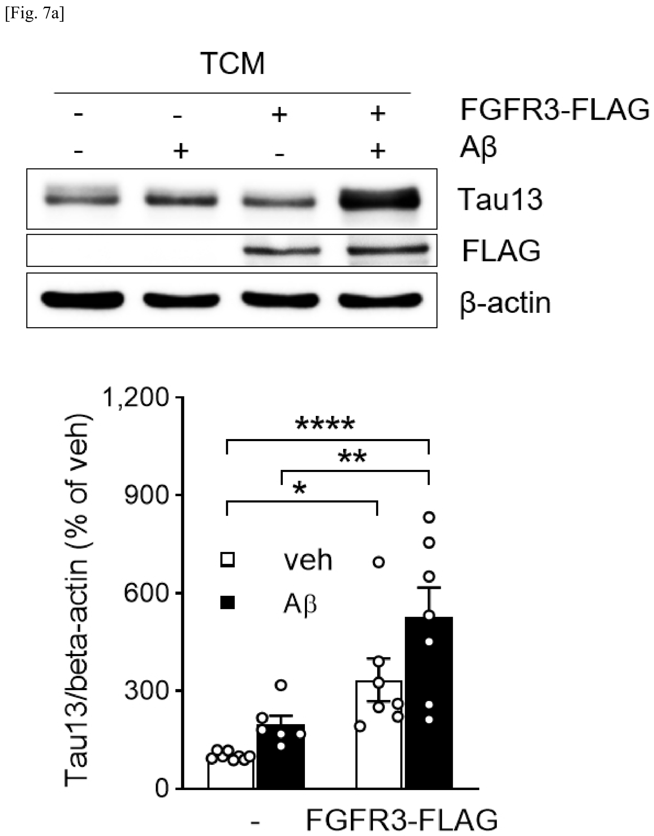

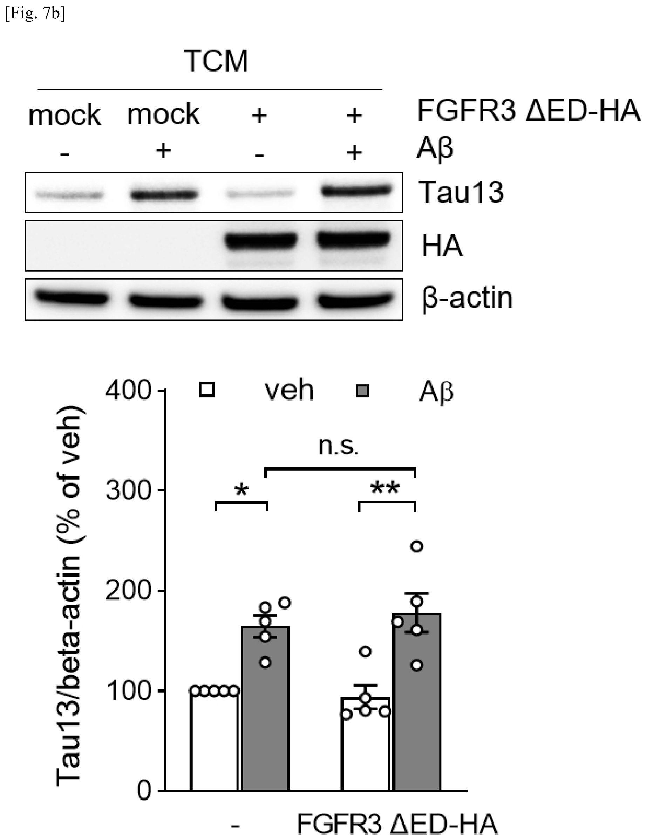

Figs. 7a and 7b show the results of expressing FLAG-tagged FGFR3 in HT22 neuronal cells through DNA transfection, observing the amount of tau absorption induced by Aβ (Fig. 7a), and observing the amount of tau absorption in cells expressing FGFR3△ED-HA DNA, a form of FGFR3 with the extracellular domain removed, through DNA transfection (Fig. 7b).

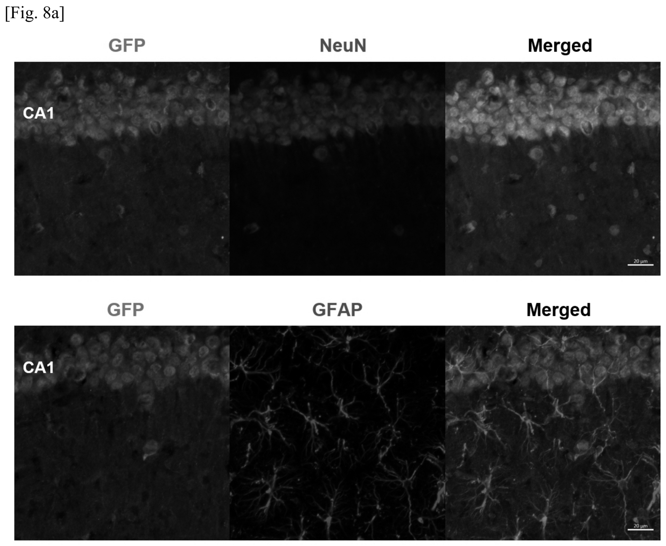

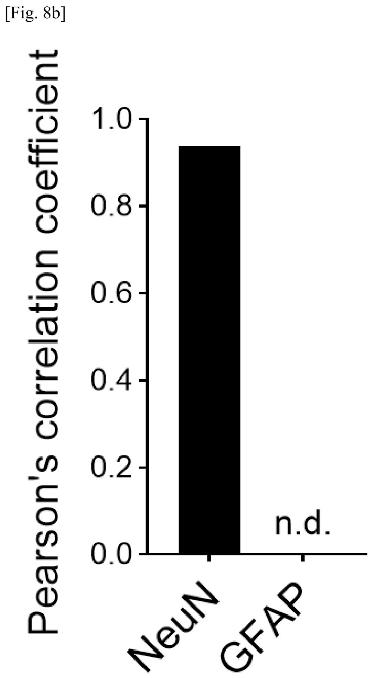

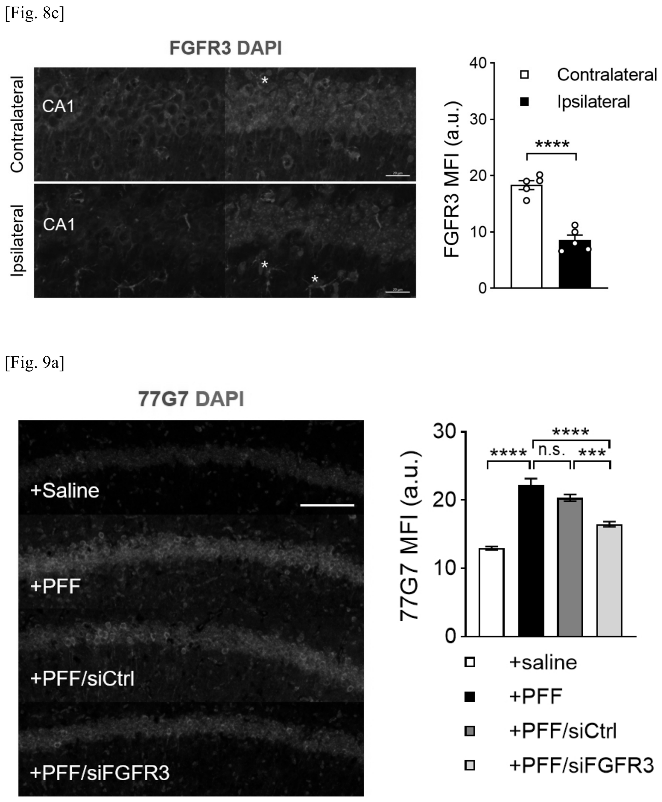

Figs. 8a to 8c show the results of producing a virus expressing siFGFR3-GFP in an AAV serotype, injecting it into the hippocampus of a mouse via stereotaxic injection, and confirming after one month that the GFP signal co-expressed with the virus appears in a pattern similar to NeuN, a marker of neuronal cells, and does not co-appear with astrocytes, the cell type where FGFR3 is expressed (Fig. 8a), quantitatively showing the colocalization of GFP and cell type markers (Fig. 8b), and quantitatively confirming that FGFR3 in neuronal cells in the hippocampus of the Alzheimer's model mouse injected with siFGFR3 is effectively reduced in expression (Fig. 8c).

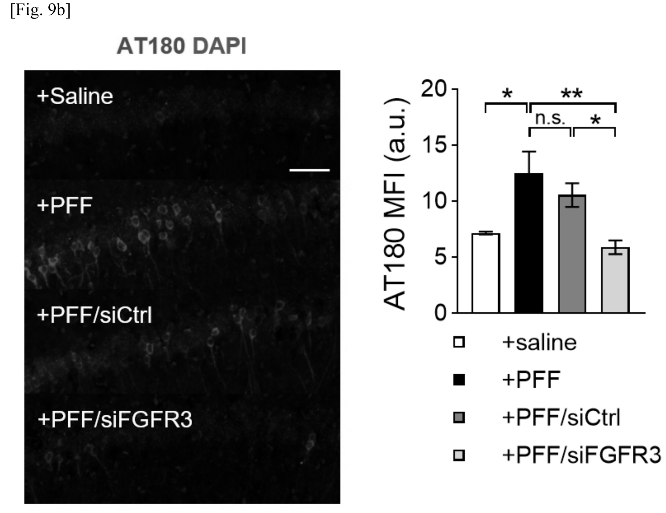

Figs. 9a and 9b show the results of observing the amount of PFF in animals where tau pathology was accelerated by additionally injecting PFF into the hippocampus of mice expressing human tau and then reducing the expression of FGFR3 by injecting siFGFR3 (Fig. 9a), and staining the hippocampus of the mouse with the AT180 antibody, which detects hyperphosphorylated tau (Fig. 9b).

### [Detailed Description of the Invention]

Hereinafter, the present invention will be described in detail with reference to the following examples. However, the following examples are merely illustrative of the present invention, and the present invention is not limited thereto.

### 1. Confirmation of the function of FGFR3 as a tau receptor accelerating tau internalization by amyloid beta

First, a culture medium of a neuronal cell line overexpressing tau protein was obtained, and the culture medium containing secreted tau was added to neuronal cells pretreated with amyloid beta (Fig. 1a). At this time, it was confirmed that the amount of tau internalized into the cells increased depending on the concentration and treatment time of amyloid beta (Fig. 1b).

To identify the receptor that actually affects internalization, vesicles were isolated from the cells, and LC-MS/MS proteomic analysis was conducted. As a result, among the receptor proteins present in the cell membrane, FGFR3 was identified as the only protein that increased in the amyloid beta-treated group compared to the control group (Figs. 2a to 2c). When the amount of FGFR3 protein in the vesicles of the cells was confirmed through western blot, it was also confirmed that the amount of FGFR3 in the neuronal cell vesicles significantly increased under the amyloid beta-pretreated condition (Fig. 2d). When it was confirmed through immunoprecipitation whether FGFR3 is indeed a tau receptor that interacts with tau protein, it was found that both tau in the culture medium of tau-overexpressing cells and the prepared tau protein physically bind to FGFR3 (Figs. 2e and 2f).

As a result, it was discovered that FGFR3, which is actively internalized by amyloid beta, directly interacts with tau and contributes to accelerating tau internalization.

### 2. Improvement of Alzheimer's disease pathology through FGFR3 expression inhibition

In an Alzheimer's disease animal model overexpressing both amyloid beta and tau protein, the expression of FGFR3 was reduced in neuronal cells by injecting siRNA viruses of SEQ ID NOs: 2 to 5, which regulate FGFR3 expression.
SEQ ID NO: 2: CCGGCCTGCGTGCTAGTGTTCTGCGTGGC
SEQ ID NO: 3: TCAGTGTGCGTGTAACAGATGCTCCATCC
SEQ ID NO: 4: CCGGAGCGAATGGATAAGAAACTGCTGGC
SEQ ID NO: 5: GCTGAGGAGGAGCTGATGGAAACTGATGA

As a result, the amount of hyperphosphorylated tau protein in the hippocampus of the mice significantly decreased (Fig. 3a), and at the same time, neuronal cell death was suppressed, resulting in an increase in the number of neuronal cells compared to the control group (Fig. 3b).

When the amount of strongly aggregated tau protein was measured through Sarkosyl-fraction, it was confirmed that the amount of aggregated tau protein insoluble in Sarkosyl significantly decreased in mice with suppressed FGFR3 gene expression (Fig. 4a). Additionally, it was confirmed that the amount of tau protein in the oligomer form, known to be harmful to neuronal cells, decreased when FGFR3 expression was inhibited (Fig. 4b).

When the memory of the mice was tested through various behavioral experiments, it was confirmed that FGFR3 regulation showed memory-enhancing effects in the Y-maze test for short-term memory (Figs. 5a and 5b), the contextual fear conditioning test (Fig. 5c) and the novel object recognition test (Figs. 5d and 5e) for long-term memory.

As a result, it was verified that inhibiting FGFR3 expression in neuronal cells is very effective in improving the pathological state of Alzheimer's disease and enhancing memory.

### 3. Reduction of tau uptake by FGFR3 expression inhibition

When siRNA targeting FGFR3 was transfected into HT22 neuronal cells, the amount of tau uptake was confirmed by protein quantification. The siRNA reduced FGFR3 protein expression by 10-20%, and the amount of tau uptake also decreased by a similar level. As a result, it was confirmed that inhibiting FGFR3 expression directly suppresses tau uptake (Figs. 6a to 6c).

### 4. Changes in tau uptake according to FGFR3 expression regulation

After expressing FLAG-tagged FGFR3 in HT22 neuronal cells through DNA transfection, the pattern of tau uptake induced by Aβ was examined. As a result, in cells with increased FGFR3 expression, tau uptake was generally higher, and this tendency was more pronounced in Aβ-treated cells (Fig. 7a). To investigate which part of FGFR3 contributes to tau uptake, FGFR3△ED-HA DNA (SEQ ID NO: 6), a form of FGFR3 with the extracellular domain removed, was transfected for expression. When this form of FGFR3 was expressed in cells, tau uptake did not increase compared to the control group. As a result, it was confirmed that the extracellular domain of FGFR3 contributes to inducing tau uptake (Fig. 7b).

[SEQ ID NO: 6]
acctgtacatgatcatgcgggaatgttggcatgcggtgccttcacagaggcccaccttcaagcagttggtagaggatttagaccgcatcctc
actgtgacatcaaccgacgagtacttggacctctccgtgccgtttgagcagtactcgccaggtggccaggacacgcctagctccagctcgt
ccggagatgactcggtgttcacccatgacctgctacccccaggtccacccagtaacgggggacctcggacggtcgagggcgtgcaggtg
gaaaccatctccccaggagacgggcgcaccttccccaagcgcggccagacctgcgtggtgcactacaccgggatgcttgaagatggaaa
gaaagttgattcctcccgggacagaaacaagccctttaagtttatgctaggcaagcaggaggtgatccgaggctgggaagaaggggttgc
ccagatgagtgtgggtcagagagccaaactgactatatctccagattatgcctatggtgccactgggcacccaggcatcatcccaccacatg
ccactctcgtcttcgatgtggagcttctaaaactggaagtcgagggagtgcaggtggaaaccatctccccaggagacgggcgcaccttccc
caagcgcggccagacctgcgtggtgcactacaccgggatgcttgaagatggaaagaaagttgattcctcccgggacagaaacaagccctt
taagtttatgctaggcaagcaggaggtgatccgaggctgggaagaaggggttgcccagatgagtgtgggtcagagagccaaactgactat
atctccagattatgcctatggtgccactgggcacccaggcatcatcccaccacatgccactctcgtcttcgatgtggagcttctaaaactggaa
tctggcggtggatccggagtcgactatccgtacgacgtaccagactacgcactcgactaa

### 5. siRNA-AAV system specifically inhibiting FGFR3 gene in neuronal cells

A virus expressing siFGFR3-GFP was produced using an AAV serotype known to be primarily expressed in neuronal cells, and it was injected into the hippocampus of mice via stereotaxic injection. One month after the injection, it was confirmed that the GFP signal co-expressed with the virus appeared in a pattern similar to NeuN, a marker of neuronal cells, and did not co-localize with astrocytes, the cell type expressing FGFR3 (Fig. 8a). The colocalization of GFP and cell type markers was quantitatively shown (Fig. 8b). It was quantitatively confirmed that FGFR3 expression in neuronal cells was effectively reduced in the hippocampus of Alzheimer's model mice injected with siFGFR3 (Fig. 8c).

### 6. Effect of FGFR3 gene inhibition on the uptake of externally injected pre-formed fibril tau (PFF)

By additionally injecting PFF into the hippocampus of mice expressing human tau, the acceleration of tau pathology was induced. At this time, the expression level of FGFR3 was reduced by co-injecting siRNA virus. When the 77G7 tau antibody, which acts on the epitope included in PFF, was used, it was confirmed that the amount of PFF in the neuronal cell layer significantly increased in the PFF-injected group, but significantly decreased in the group where FGFR3 expression was reduced by siFGFR3 injection (Fig. 9a). When the hippocampus of mice was stained with the AT180 antibody, which detects hyperphosphorylated tau, the PFF-injected group showed significantly higher levels of tau pathology in neuronal cells compared to the control group due to the effect of PFF uptake. On the other hand, when siFGFR3 was co-injected to reduce the amount of FGFR3 in neuronal cells, the progression of such tau pathology was suppressed (Fig. 9b).

### [Industrial Applicability]

The present invention reveals that FGFR3 can be used as a new target for the treatment of neurodegenerative diseases, and based on this, provides a preventive and therapeutic agent for neurodegenerative diseases. In addition, it provides a method for screening candidate substances for the prevention and treatment of neurodegenerative diseases. According to the screening method of the present invention, new candidate substances capable of preventing and/or treating neurodegenerative diseases can be effectively selected, and by using the substances selected according to the screening method of the present invention, it is possible to provide a pharmaceutical composition for the prevention and treatment of neurodegenerative diseases, thereby having high industrial applicability.

## Claims

1. A pharmaceutical composition for preventing or treating a neurodegenerative disease, comprising an inhibitor of expression or activity of FGFR3 (fibroblast growth factor receptor 3) as an active ingredient.

2. The pharmaceutical composition according to claim 1, wherein the neurodegenerative disease is mediated by tau protein.

3. The pharmaceutical composition according to claim 1, wherein the inhibition of expression or activity of FGFR3 is in neuronal cells.

4. The pharmaceutical composition according to claim 1, wherein the neurodegenerative disease is Alzheimer's disease, frontotemporal dementia, frontotemporal lobar degeneration, corticobasal degeneration, progressive supranuclear palsy, Pick's disease, Parkinson's disease or Huntington's disease.

5. The pharmaceutical composition according to claim 1, wherein the inhibitor of FGFR3 expression is siRNA (small interference RNA), shRNA (short hairpin RNA), miRNA (microRNA), ribozyme, DNAzyme, PNA (peptide nucleic acids), antisense oligonucleotide or aptamer that complementarily binds to DNA or mRNA of FGFR3 gene.

6. The pharmaceutical composition according to claim 1, wherein the inhibitor of FGFR3 activity is a compound, peptide, peptide mimetics, fusion protein, antibody or aptamer that specifically binds to FGFR3 protein.

7. The pharmaceutical composition according to any one of claims 1 to 6, wherein the inhibitor of expression or activity of FGFR3 is an inhibitor of expression or activity of the extracellular domain of FGFR3.

8. A method for screening a candidate substance for preventing and treating a neurodegenerative disease, comprising the steps of:
(a) treating a test substance to cells expressing mRNA or protein of FGFR3;
(b) measuring the expression level of FGFR3 mRNA, the expression level of FGFR3 protein, or the activity of FGFR3 protein; and
(c) selecting a substance that decreases the expression level of FGFR3 mRNA, the expression level of FGFR3 protein, or the activity of FGFR3 protein compared to a control group (untreated group) as a candidate substance.

9. The method according to claim 8, wherein the measurement of the expression level of FGFR3 mRNA is performed using a method selected from the group consisting of RT-PCR, quantitative or semi-quantitative RT-PCR, quantitative or semi-quantitative real-time RT-PCR, northern blot, and DNA or RNA chip.

10. The method according to claim 8, wherein the measurement of the expression level of FGFR3 protein is performed using a method selected from the group consisting of western blot, ELISA, radioimmunoassay, radial immunodiffusion, Ouchterlony immunodiffusion, rocket immunoelectrophoresis, immunohistochemical staining, immunoprecipitation analysis, complement fixation analysis, FACS and protein chip.

11. A food composition for preventing or improving a neurodegenerative disease, comprising an inhibitor of expression or activity of FGFR3 (fibroblast growth factor receptor 3) as an active ingredient.

12. Use of an inhibitor of expression or activity of FGFR3 (fibroblast growth factor receptor 3) for manufacturing a composition for treating a neurodegenerative disease.

13. A method for treating a neurodegenerative disease, comprising administering a composition comprising an inhibitor of expression or activity of FGFR3 (fibroblast growth factor receptor 3) as an active ingredient to a subject in need thereof.
